# EUROPÄISCHE PATENTSCHRIFT

(11) **EP 3 308 692 B1**
(45) Veröffentlichungstag und Bekanntmachung des Hinweises auf die Patenterteilung: **08.07.2020**
(21) Anmeldenummer: 17194061.2
(22) Anmeldetag: 29.09.2017
(51) Int. Cl.: A61B 1/12, B29C 64/20, B29C 64/393, B29L 31/00, A61B 90/00, A61B 90/70, A61B 1/00, B08B 9/00

(54) **ADAPTER ZUM ANSCHLUSS EINES ENDOSKOPS, SYSTEM UND VERFAHREN ZUR HERSTELLUNG EINES ADAPTERS**
ADAPTER FOR CONNECTING AN ENDOSCOPE, SYSTEM AND METHOD FOR PRODUCING ADAPTOR
ADAPTEUR PERMETTANT DE CONNECTER UN ENDOSCOPE, SYSTÈME ET PROCÉDÉ DE FABRICATION D'UN ADAPTEUR

(30) Priorität: 17.10.2016 DE 102016119707
(43) Veröffentlichungstag der Anmeldung: 18.04.2018
(73) Patentinhaber: OLYMPUS WINTER & IBE GMBH, 22045 Hamburg (DE)
(72) Erfinder: Boettcher-Wilmes, Marius, 23858 Reinfeld (DE)
(74) Vertreter: Noack, Andreas

(56) Entgegenhaltungen:
- DE-A1-102012 218 811
- DE-A1-102014 220 902
- DE-B4-102012 020 934
- US-A1- 2013 329 257
- US-A1- 2015 173 593
- US-A1- 2016 193 012

## Beschreibung

Die Erfindung betrifft einen Adapter zum Anschluss eines Endoskops mit wenigstens einem Endoskopkanal an ein Aufbereitungsgerät mit wenigstens einem Aufbereitungskanal, wobei der Adapter ein Verbindungskanalsystem aufweist, welches zur Verbindung des wenigstens einen Endoskopkanals mit dem wenigstens einen Aufbereitungskanal vorgesehen ist, um eine zuverlässige Aufbereitung des wenigstens einen Endoskopkanals durch das Aufbereitungsgerät zu ermöglichen.

Endoskope werden seit längerer Zeit besonders in der Medizin eingesetzt, um schwer zugängliche Regionen des menschlichen Körpers zu untersuchen und ggf. zu behandeln. Dazu weisen Endoskope einen langgestreckten Endoskopschaft auf, welcher über natürliche oder chirurgisch erzeugte Öffnungen in den Körper des Patienten eingeführt und bis in die interessierende Region vorgeschoben wird. Der Endoskopschaft kann dabei starr oder flexibel sein.

Im Inneren des Endoskopschafts sind in der Regel ein oder mehrere Kanäle vorgesehen, durch welche während der Untersuchung bzw. des Eingriffs Flüssigkeiten zu- oder abgeführt werden, oder welche zum Einbringen verschiedener Instrumente dienen.

Nach der Verwendung eines Endoskops muss dieses sorgfältig aufbereitet werden, bevor eine erneute Verwendung möglich ist. Dies erfolgt in mehreren Schritten, wobei zunächst in einer meist manuellen Vorreinigung die Endoskopkanäle mittels Bürsten gereinigt werden, anschließend wird das Endoskop in einem automatischen Aufbereitungsgerät einem weiteren Reinigungsprozess sowie einem Desinfektionsprozess und einem Trocknungsprozess unterzogen. Als Aufbereitungsgeräte können z.B. Maschinen der Serie ETD der Anmelderin verwendet werden.

Ein wesentlicher Bestandteil von Aufbereitungsgeräten ist ein Spülsystem zum Durchspülen der Endoskopkanäle. Das Spülsystem weist in der Regel einen oder mehrere Aufbereitungskanäle auf, durch welche Spül-, Reinigungs- und/oder Desinfektionsflüssigkeiten zu den Endoskopkanälen geführt werden können. Die einzelnen Aufbereitungskanäle weisen dabei separate oder gemeinsame Pumpen, Ventile und Sensoren auf, mittels welcher die Flüssigkeitsströme erzeugt, gesteuert und überwacht werden.

Für unterschiedliche medizinische Anwendungen sind eine Vielzahl unterschiedlicher Endoskoptypen bekannt, welche sich in Anzahl, Art und Struktur der Endoskopkanäle stark unterscheiden. Um trotzdem eine Vielzahl von, und bestenfalls alle, Endoskoptypen aufbereiten zu können, werden Adapter eingesetzt, um die Endoskopkanäle eines aufzubereitenden Endoskops mit den Aufbereitungskanälen eines Aufbereitungsgeräts zu verbinden.

Die DE102012020934 A1 beschreibt ein Verfahren zum Reinigen und Desinfizieren von Endoskopen unter Einsatz eines gattungsgemäßen Adapters.

Die DE102014220902A1 beschreibt eine Anschlussvorrichtung, mittels welcher medizintechnische Geräte schnell mit verschiedenen elektrischen Leitungen und Fluidleitungen verbunden und von diesen wieder getrennt werden können. Die Anschlussvorrichtung weist mehrere Anschlussmodule auf, die in einem 3D-Druckverfahren hergestellt sein können.

Die US2013/0329257A1 beschreibt ein System zum bereitstellen von 3D-Druckmodellen über ein Netzwerk.

Die US2015/0173593A1 beschreibt ein Endoskop mit einem internen Element zum Verbinden mechanischer Komponenten, welches im 3D-Druckverfahren hergestellt sein kann.

Die US2016/0193012A1 beschreibt ein Reinigungsgerät für medizinische Geräte. Das Reinigungsgerät umfasst eine Spülkammer, welche in einem 3D-Druckverfahren hergestellt sein kann.

In der Regel für ist jeden Endoskoptyp und jedes Aufbereitungsgerät ein Separater Adapter vorgesehen, so dass beispielsweise in einem Krankenhaus, in welchem viele unterschiedliche Endoskoptypen eingesetzt werden, auch viele unterschiedliche Adapter vorgehalten werden müssen.

Bei der Verwendung der Adapter zur Aufbereitung von Endoskopen kann durch Auswahl eines falschen Adapters das Aufbereitungsergebnis negativ beeinflusst werden. Weiterhin kann das Aufbereitungsergebnis negativ beeinflusst werden, wenn der verwendete Adapter abgenutzt, beschädigt oder durch unsachgemäße Lagerung kontaminiert ist.

Es besteht daher die Aufgabe der Erfindung darin, Adapter und Systeme zur Aufbereitung von Endoskopen bereitzustellen, welche hinsichtlich der beschriebenen Problematik verbessert sind.

Diese Aufgabe wird gemäß der Erfindung gelöst durch einen Adapter gemäß Anspruch 1, einem System gemäß Anspruch 6 und einem Verfahren gemäß Anspruch 10. Die abhängigen Ansprüche beziehen auf bevorzugte Ausführungen der Erfindung.

Aspekte, Ausführungen, Ausgestaltungen und Beispiele dieser Beschreibung, die nicht unter den Wortlaut der beigefügten Ansprüche fallen, sind nicht Teil der Erfindung.

Die Herstellung des Adapters in einem 3D-Durchverfahren ermöglicht die Herstellung des Adapters ,on demand', also erst dann, wenn der Adapter tatsächlich für die Aufbereitung eines Endoskops benötigt wird. Hierdurch lassen sich lange Lagerzeiten der Adapter und damit verbundene Probleme vermeiden.

In einer Ausführung der Erfindung weist ein Adapter einen das Verbindungskanalsystem umfassenden Hauptkörper auf, und der Hauptkörper ist in einem 3D- Druckverfahren hergestellt.

Zusätzlich zu dem Hauptkörper kann ein Adapter weitere Komponenten beinhalten, welche nicht für einzelne Endoskoptypen spezifisch sind. Solche unspezifischen Komponenten können unproblematisch bevorratet werden, so dass eine Herstellung ,on demand' nicht erforderlich ist.

In einer möglichen Ausgestaltung der Erfindung weist ein Adapter Schlauchkupplungen auf, und die Schlauchkupplungen sind nicht in einem 3D-Druckverfahren hergestellt.

In einer weiteren möglichen Ausgestaltung der Erfindung umfasst der Adapter Schläuche, und die Schläuche sind nicht in einem 3D-Druckverfahren hergestellt.

Die Herstellung des Adapters kann beispielsweise in einem einfachen 3D-Drucker erfolgen, wobei nicht in einem 3D-Druckverfahren hergestellte Komponenten nachträglich manuell montiert werden. Alternativ kann die Herstellung des Adapters vollständig in einer kombinierten Anlage erfolgen, welche beispielsweise zunächst den 3D-Druck durchführt und anschließend zusätzliche Komponenten wie Schlauchkupplungen oder Schläuche mittels eines Montageroboters montiert.

Gemäß der Erfindung ist der Adapter zur Einmalverwendung vorgesehen. Der Adapter wird also nach der einmaligen Verwendung entsorgt. Hierdurch kann eine Lagerhaltung von Adaptern und die damit verbundenen Kosten und Risiken ganz entfallen.

Um eine versehentliche oder vorsätzliche Wiederverwendung eines bereits verwendeten Adapters zu verhindern weist gemäß der Erfindung der Adapter ein Kupplungselement zur Verbindung mit dem Aufbereitungsgerät auf, welches zum Lösen des Adapters von dem Aufbereitungsgerät zerstört werden muss.

Beispielsweise kann der Adapter eine oder mehrere Rastzungen aufweisen, mit welchen der Adapter in einer vorgesehenen Position in dem Aufbereitungsgerät gehalten wird. Diese Rastzungen können so angeordnet sein, dass sie beim Trennen des Adapters vom dem Aufbereitungsgerät brechen und somit eine Wiederverwendung verhindern.

Gemäß eines gesonderten Aspekts der Erfindung kann ein Adapter zumindest teilweise aus einem wiederverwendbaren Druckmaterial hergestellt sein. So kann der Materialverbrauch zur Herstellung der Adapter verringert werden, wenn Material der Adapter nach deren Verwendung wiederverwendet wird. Dazu können Adapter nach ihrer Verwendung zerkleinert und in wiederverwendbares und nicht wiederverwendbares Material getrennt werden. Das wiederverwendbare Material wird dann aufgereinigt und zur Herstellung neuer Adapter verwendet.

Die Aufgabe der Erfindung wird weiterhin gelöst durch ein System gemäß Anspruch 6.

Mittels des erfindungsgemäßen Systems kann ein aufzubereitendes Endoskop automatisch ermittelt und identifiziert werden. Die Steuerung kann dann ein vom Benutzer ausgewähltes Aufbereitungsgerät ermitteln und mittels der in der Datenbank vorgehaltenen Druckdaten einen entsprechenden Adapter herstellen. Falls das System mehrere Aufbereitungsgeräte umfasst, kann die Steuerung auf Basis der vorliegenden Daten auch eines der Aufbereitungsgeräte zur Auswahl vorschlagen oder die Auswahl des zu verwendenden Aufbereitungsgeräts automatisch vornehmen.

Dabei kann das 3D-Drucksystem außer einem 3D-Drucker weitere Elemente aufweisen, welche auch die Herstellung bzw. Montage von nicht in 3D-Druckverfahren hergestellten Komponenten eines Adapters ermöglichen.

Ebenso kann die Datenbank neben 3D-Druckdaten weitere Daten bezüglich der Herstellung von Adaptern beinhalten, welche sich auf nicht in 3D-Druckverfahren hergestellte Komponenten des Adapters beziehen.

In einer möglichen Ausgestaltung eines Systems gemäß der Erfindung ist der Detektor ausgeführt, ein aufzubereitendes Endoskop zu identifizieren, wenn dieses in ein Aufbereitungsgerät eingebracht wird. Hierzu kann ein üblicherweise in Aufbereitungsgeräten vorgesehener RFID-Scanner oder ein Barcodeleser eingesetzt werden.

In der vorgenannten Ausgestaltung kann sich der Schritt der Auswahl eines zur Aufbereitung des Endoskops verfügbaren Aufbereitungsgeräts auf eine Prüfung beschränken, ob das Aufbereitungsgerät, in welchem das aufzubereitende Endoskop erkannt wurde, zur Aufbereitung des entsprechenden Endoskoptyps überhaupt geeignet ist.

In einer alternativen Ausgestaltung eines Systems gemäß der Erfindung ist der Detektor ausgeführt, ein aufzubereitendes Endoskop zu identifizieren, sobald dieses Endoskop in einer Prozedur verwendet wird. So kann beispielsweise ein von dem Endoskop an eine Videoeinheit gesendeter Identifikationscode ausgewertet werden, um das verwendete Endoskop zu identifizieren.

In einer besonderen Ausführung eines erfindungsgemäßen Systems kann dieses weiterhin eine Einrichtung zur Rückgewinnung von 3D-Druckmaterial aus gebrauchten Adaptern umfassen. Eine solche Einrichtung kann mehrere Einheiten zum Zerkleinern von verwendeten Adaptern, zum Trennen der Materialien, zum Aufreinigen wiederverwendbarer Materialien sowie zum Bereitstellen der aufgereinigten Materialien in einer für die Zuführung zu einem 3D-Druckverfahren geeigneten Form, umfassen.

Die Aufgabe wird weiterhin gelöst durch ein Verfahren gemäß Anspruch 10.

Bezüglich weiterer möglicher Ausgestaltungen des erfindungsgemäßen Verfahrens und der dadurch erreichten Vorteile wird ausdrücklich auf die obige Beschreibung verwiesen.

Zur Herstellung des Adapters wird ein Kunststoff verwendet, zum Beispiel Polyactide (PLA) und/oder Acrylnitril-Butadien-Styrol (ABS).

Die Erfindung wird nachfolgend anhand einiger stark vereinfachter Zeichnungen näher erläutert. Es zeigen:
- Fig.1: Ein Endoskop mit einem zugeordneten Adapter in schematischer Ansicht,
- Fig.2: einen Adapter in einer Schnittdarstellung,
- Fig.3: ein System zur Aufbereitung von Endoskopen in einer ersten Variante,
- Fig.4: ein System zur Aufbereitung von Endoskopen in einer zweiten Variante,
- Fig.5: ein System zur Aufbereitung von Endoskopen in einer dritten Variante.

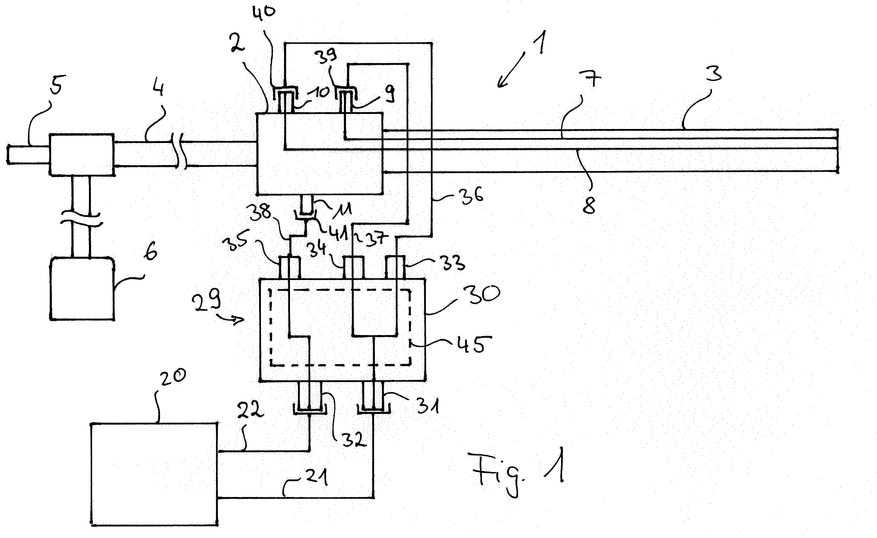

In Figur 1 ist schematisch ein Endoskop 1 mit einem Endoskophauptkörper 2 und einem Endoskopschaft 3 dargestellt. Am proximalen Ende des Endoskophauptkörpers 2 ist ein Anschlusskabel 4 vorgesehen, welches ein einem Lichtleitstecker 5 und einem Videostecker 6 endet.

Im Inneren des Endoskops 1 sind Kanäle 7,8 vorgesehen, durch welche Flüssigkeiten und/oder endoskopische Instrumente geführt werden können. Der Kanal 7 mündet in einem Anschlussstutzen 9 am Endoskophauptkörper 2, der Kanal 8 mündet in einem Anschlussstutzen 10 am Endoskophauptkörper.

Ein weiterer Anschlussstutzen 11 am Endoskophauptkörper ist mit dem Innenraum des Endoskops 1 verbunden und wird während der Aufbereitung dazu genutzt, die Dichtheit des Endoskops gegen Eindringen von Flüssigkeiten zu prüfen.

Nach der Verwendung des Endoskops 1 muss dieses aufbereitet werden. Dazu wird das Endoskop 1 nach einer zumeist manuellen Vorreinigung in ein hier nicht dargestelltes Aufbereitungsgerät eingelegt, in welchem die Dichtheit des Endoskops 1 geprüft und die Kanäle 7,8 mit Spül-, Reinigungs- und/oder Desinfektionsflüssigkeit durchspült werden. Dazu weis das Aufbereitungsgerät eine Spüleinheit 20 auf, welche im dargestellten Beispiel zwei Aufbereitungskanäle 21,22 aufweist, aber auch mehr oder weniger Kanäle aufweisen kann.

Im dargestellten Beispiel dient der Aufbereitungskanal 21 zum Spülen von Endoskopkanälen, während der Aufbereitungskanal 22 zur Dichtheitsprüfung von Endoskopen dient.

Um das Endoskop 1 aufzubereiten, muss der Aufbereitungskanal 21 der Spüleinheit 20 über die Anschlussstutzen 9,10 mit den Kanälen 7,8 des Endoskops 1 verbunden werden. Zusätzlich muss der Aufbereitungskanal 22 mit dem Anschlussstutzen 11 des Endoskops 1 verbunden werden.

Die Verbindung der Aufbereitungskanäle 21,22 mit den Anschlussstutzen 9,10,11 erfolgt über einen Adapter 29. Der Adapter 29 weist einen Hauptkörper 30 mit zwei Eingangsstutzen 31,32 auf, welche mit den Aufbereitungskanälen 21,22 verbunden werden können. Weiterhin weist der Hauptkörper 30 drei Ausgangsstutzen 33,34,35 auf, an welchen Schläuche 36,37,38 befestigt sind. Die Schläuche 36,37,38 sind endseitig mit Schlauchkupplungen 39,40,41 versehen, welche auf die Anschlussstutzen 9,10,11 des Endoskops aufgesetzt werden können.

Die Anschlussstutzen 9,10,11 des Endoskops 1 können dabei unterschiedliche Formate aufweisen, daher sind die Schlauchkupplungen 39,40,41 spezifisch für das jeweilige Endoskop 1 ausgelegt.

Im Inneren des Hauptkörpers 30 ist ein Kanalsystem 45 vorgesehen, welches die Eingangsstutzen 31,32 so mit den Ausgangsstutzen 33,34,35 verbindet, dass bei korrektem Anschluss aller Verbindungen eine sichere Aufbereitung des Endoskops 1 ermöglicht ist.

Um den Adapter 29 ohne aufwendige Lagerhaltung bei Bedarf schnell bereitstellen zu können, wird dieser zumindest teilweise bei Bedarf in einem 3D-Druckverfahren hergestellt.

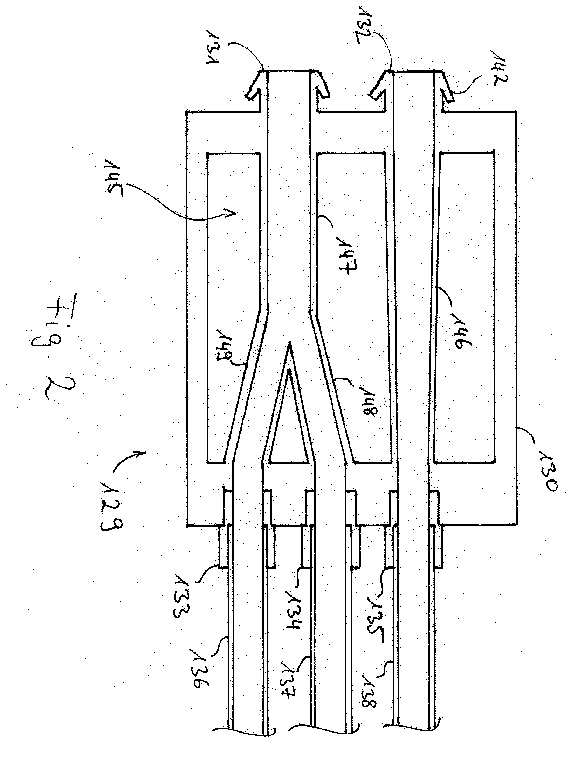

Figur 2 zeigt einen Adapter 129 in einer Schnittdarstellung. Der Adapter 129 besteht wiederum aus einem Hauptkörper 130 mit Eingangsstutzen 131,132 und Ausgangsstutzen 133,134,135. An den Ausgangsstutzen 133,134,135 sind Schläuche 136,137,138 angeordnet. Dazu sind die Ausgangsstutzen als Klemmhülsen ausgeführt, welche in den Hauptkörper 130 eingeschraubt sind, und in welchen die Schläuche 136,137,138 durch einfaches Einschieben unlösbar befestigt werden.

Im Gegensatz zu den Ausgangsstutzen 133,134,135 sind die Eingangsstutzen 131,132 einstückig mit dem Hauptkörper 130 in einem 3D-Druckverfahren hergestellt.

Im Inneren des Hauptkörpers 130 ist ein Kanalsystem 145 vorgesehen, welches die Eingangsstutzen 131,132 mit den Ausgangsstutzen 133,134,135 verbindet. Das Kanalsystem 145 besteht aus röhrenförmigen Kanälen 146,147,148,149, welche ebenfalls einstückig mit dem Hauptkörper in einem 3D-Druckverfahren hergestellt sind.

Der Adapter 129 ist zur Einmalverwendung vorgesehen, um durch Lagerung und Verschleiß bedingte Probleme bei der Aufbereitung von Endoskopen zu vermeiden. Um eine bewusste oder unbewusste Mehrfachverswendung des Adapters 129 zu verhindern, sind an den Eingangsstutzen 131,132 Rastnasen 142 angeformt, welche beim Anschluss des Adapters 129 an Aufbereitungskanäle eines Aufbereitungsgeräts in Hinterschneidungen der komplementären Anschlusselemente einrasten. Beim Lösen des Adapters 129 von dem Aufbereitungsgerät müssen die Rastnasen 142 gebrochen werden, eine erneute Verbindung ist danach nicht mehr möglich.

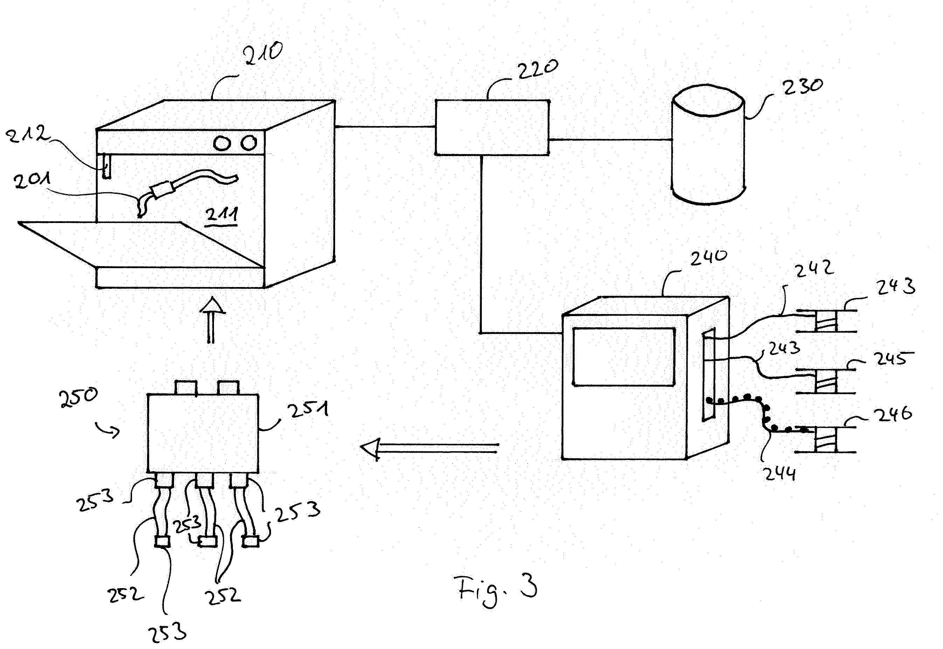

Figur 3 zeigt ein System zur Aufbereitung eines Endoskops 201. Das System umfasst ein Aufbereitungsgerät 210, in dessen Reinigungsraum 211 das Endoskop 201 zur Aufbereitung eingebracht wird. Ein im Bereich des Reinigungsraums 211 angeordneter Detektor 212 erkennt und identifiziert das Endoskop 201. Dazu ist der Detektor 212 beispielsweise als RFID-Scanner ausgeführt, welcher einen nicht dargestellten, am Endoskop 201 angebrachten RFID-Chip erkennt. Der Detektor 212 kann wie dargestellt in dem Aufbereitungsgerät 210 integriert sein. Alternativ kann der Detektor 212 als externer Handscanner ausgeführt sein, welcher per Kabel oder Funkverbindung mit dem Aufbereitungsgerät 210 verbunden ist. Das Aufbereitungsgerät nutzt die vom Detektor 212 ermittelte Information, um ein für die Aufbereitung des Endoskops 201 geeignetes Aufbereitungsprogramm auszuwählen.

Das System ist umfasst weiterhin eine Steuerung 220, eine Datenbank 230 sowie ein 3D-Drucksystem 240. In der Datenbank 230 sind 3D-Druckdaten verschiedener Adapter hinterlegt, mittels welcher das 3D-Drucksystem 240 diese Adapter herstellen kann. Weiterhin umfasst die Datenbank 230 Informationen darüber, für welche Endoskoptypen und Aufbereitungsgeräte der jeweilige Adapter geeignet ist.

Die Steuerung 220 erhält von dem Aufbereitungsgerät 210 Informationen über das vom Detektor 212 ermittelte Endoskop 201, insbesondere über den Endoskoptyp, sowie den Typ des Aufbereitungsgeräts 210 selbst. Auf Basis dieser Informationen ermittelt die Steuerung 220 aus der Datenbank 230 einen geeigneten Adapter sowie die entsprechenden 3D-Druckdaten.

Die Steuerung 220 überträgt die ermittelten 3D-Druckdaten an das 3D-Drucksystem 240, welches den Adapter 250 zumindest teilweise in einem 3D-Druckverfahren herstellt.

Dazu verwendet das 3D-Drucksystem ein geeignetes Material, z.B. einen Kunststoff, welcher in Form eines Filaments 241 von einer Spule 242 zugeführt werden kann. Andere Arten der Materialzufuhr, z.B. in Form eines Granulats, sind ebenfalls denkbar. Als Materialien kommen beispielsweise Polyactide (PLA), Acrylnitril-Butadien-Styrol (ABS) oder vergleichbare Kunststoffe in Frage.

Der Adapter 250 kann außer einem im 3D-Druckverfahren herzustellenden Hauptkörper 251 weitere Komponenten aufweisen, welche nicht oder nicht vorteilhaft in einem 3D-Druckverfahren herzustellen sind. Hierbei kann es sich um Schlauchabschnitte 252 und Schlauchkupplungen 253 handeln. Entsprechende Komponenten können nach dem Abschluss des 3D-Druckverfahrens manuell mit dem Hauptkörper 251 verbunden werden. Alternativ kann das 3D-Drucksystem 240 außer einer 3D-Druckeinheit weitere Fertigungskomponenten, z.B. robotische Manipulatoren, umfassen, welche die zusätzlichen Komponenten 252,253 automatisch mit dem Hauptkörper 251 verbinden. Dazu kann das entsprechende Material als Endlosmaterial 243 oder Bestückungsstreifen 244 ebenfalls auf Spulen 245,246 bereitgestellt werden.

Je nach Komplexität des Adapters 250 kann dieser in wenigen Minuten, bei weiterem Fortschritt der 3D-Drucktechnologie auch in wenigen Sekunden, nach Identifikation des Endoskops 201 fertiggestellt sein. Der Adapter wird dann verwendet, um die Endoskopkanäle des Endoskops 201 mit den Aufbereitungskanälen des Aufbereitungsgeräts 210 zu verbinden, um die sichere Aufbereitung des Endoskops zu ermöglichen.

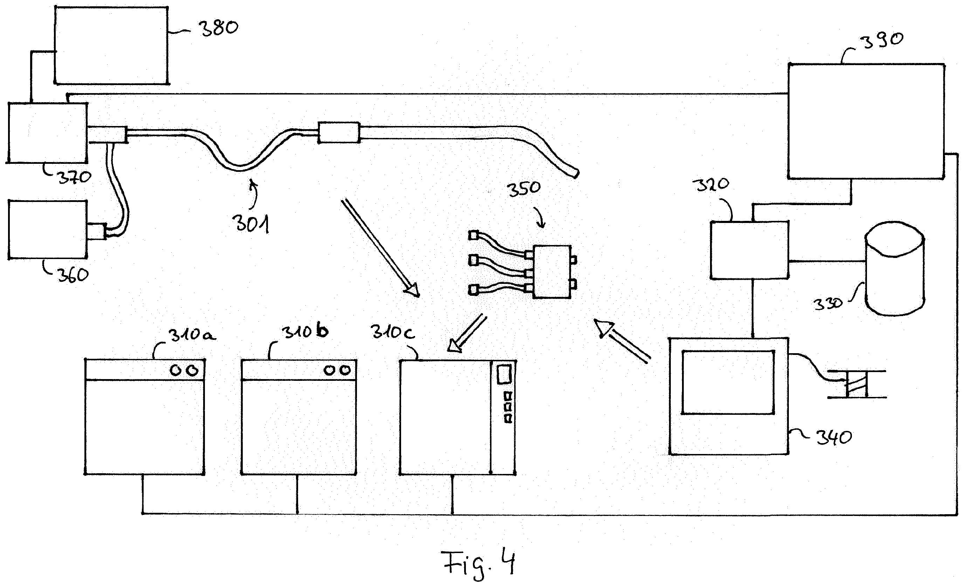

Figur 4 zeigt ein alternatives System zur Aufbereitung eines Endoskops 301. Im Gegensatz zu dem in Figur 3 gezeigten System sind hier beispielhaft drei Aufbereitungsgeräte 310a,310b,310c vorgesehen, wobei das Aufbereitungsgerät 310c von einem anderen Typ ist als die Aufbereitungsgeräte 310a,310b. Das in Figur 4 dargestellte System umfasst ebenfalls eine Steuerung 320, eine Datenbank 330 sowie ein 3D-Drucksystem 340 zur Herstellung eines Adapters 350, welche in Funktion und Aufbau im Wesentlichen mit den entsprechenden Elementen des in Figur 3 gezeigten Systems übereinstimmen.

Anders als das in Figur 3 dargestellte System ist das in Figur 4 dargestellte System eingerichtet, das aufzubereitende Endoskop 301 bereits zu identifizieren, sobald dieses in einer medizinischen Prozedur verwendet wird.

Zur Verwendung in einer medizinischen Prozedur wird das Endoskop 301 mit einer Lichtquelle 360 und mit einer Videoeinheit 370 verbunden. Die Videoeinheit 370 verarbeitet ein von dem Endoskop 301 geliefertes Videobild zur Darstellung auf einem Monitor 380. Dabei übermittelt das Endoskop 301 der Videoeinheit 370 Identifikationsdaten, welche von der Videoeinheit 370 an ein Endoskop-Verwaltungssystem 390 weitergeleitet werden.

Das Endoskop-Verwaltungssystem verwendet die Identifikationsdaten, um die Verwendung des Endoskops 301 zu dokumentieren. Dabei können verschiedene Verwendungsdaten erfasst werden, wie beispielsweise Art der durchgeführten Prozeduren, behandelte bzw. untersuchte Patienten, verwendende Ärzte, Aufbereitungsprotokolle oder aufgetretene Störungen. Als Endoskop-Verwaltungssystem 390 kann beispielsweise das Produkt endobase der Anmelderin zum Einsatz kommen.

Das Endoskop-Verwaltungssystem 390 ist weiter eingerichtet, die Information über die Verwendung des Endoskops 301 und dessen Identifizierung an die Steuerung 320 weiterzuleiten. Somit übernimmt das Endoskop-Verwaltungssystem 390 gemeinsam mit der Videoeinheit 370 die Funktion des Detektors.

Die Steuerung 320 greift dann auf die Datenbank 330 zu, um zu ermitteln, in welchen der Aufbereitungsgeräte 310a,310b,310c das Endoskop 301 aufbereitet werden kann. Dabei können neben der Kompatibilität der Aufbereitungsgeräte 310a,310b,310c mit dem Endoskop 301 auch die derzeitigen Betriebszustände und/oder Arbeitspläne der Aufbereitungsgeräte 310a,310b,310c berücksichtigt werden, welche durch das Endoskop-Verwaltungssystem 390 bereitgestellt werden. So kann die Steuerung 320 ein Aufbereitungsgerät ermitteln, welches Aufbereitungsgerät gerade frei sein wird, wenn die derzeit mit dem Endoskop 301 durchgeführte Prozedur voraussichtlich beendet ist.

Die Steuerung ermittelt dann die 3D-Druckdaten des benötigten Adapters 350 aus der Datenbank 330 und gib diese an das 3D-Drucksystem 340, welche den entsprechenden Adapter 350 herstellt.

Da mit der Herstellung des Adapters 350 im beschriebenen Ausführungsbeispiel schon begonnen werden kann, sobald das aufzubereitende Endoskop 301 in einer Prozedur verwendet wird, kann der Adapter 350 selbst bei einem relativ langsamen Herstellungsprozess mit derzeit verfügbarer 3D-Drucktechnologie rechtzeitig fertiggestellt werden, damit das Endoskop 301 nach der Prozedur sofort aufbereitet werden kann und für eine weitere Prozedur zur Verfügung steht.

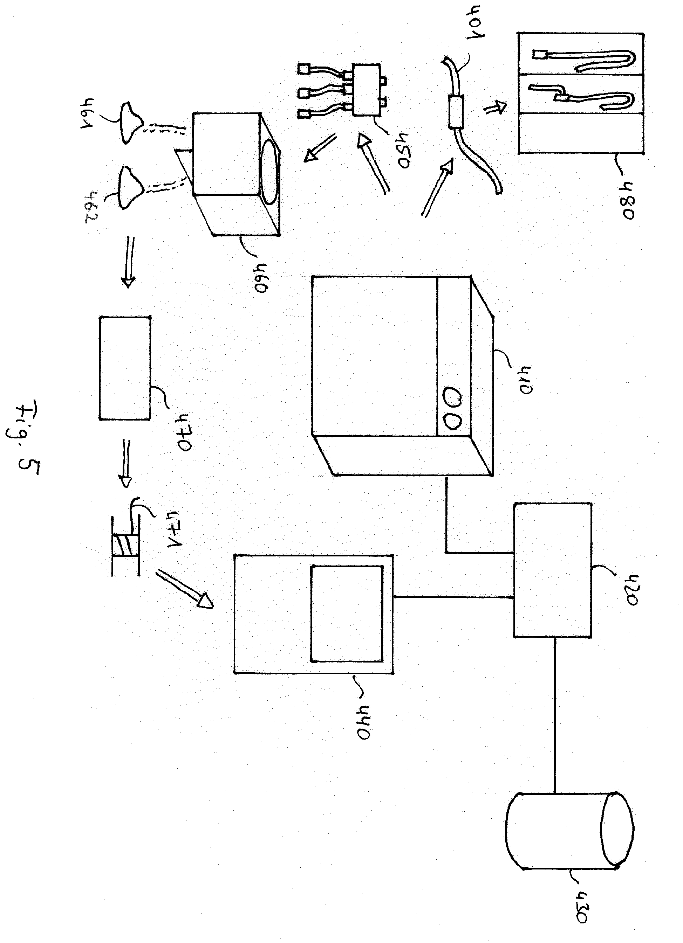

In Figur 5 ist ein weiteres System zur Aufbereitung eines Endoskops 401 dargestellt, welches ein Aufbereitungsgerät 410, eine Steuerung 420, eine Datenbank 430 sowie ein 3D-Drucksystem 440.

Das hier gezeigte System umfasst zusätzlich eine Einrichtung zur Rückgewinnung von 3D-Druckmaterial aus gebrachten Adaptern 450 mit einer Zerkleinerungs- und Fraktioniervorrichtung 460 sowie einer Konditioniervorrichtung 470.

Nach der vollständigen Aufbereitung des Endoskops 401 wird dieses mit dem Adapter 450 aus dem Aufbereitungsgerät 410 entnommen und von dem Adapter 450 getrennt.

Das Endoskop wird dann in eine Trocknungs- und Lagereinrichtung 480 eingebracht, wo es bis zur nächsten Verwendung aufbewahrt wird.

Der Adapter 450 wird in die Zerkleinerungs- und Fraktioniervorrichtung 460 verbracht, wo er beispielsweise durch ein Mahlwerk zerkleinert wird. Das Zerkleinerte Material wird in eine nicht wiederverwendbare Fraktion 461 und eine wiederverwendbare Fraktion 462 aufgetrennt, hierzu können bekannte Sortiertechniken für Schüttgut angewendet werden, welche die Fraktionen z.B. auf Basis optischer, gravimetrischer oder dielektrischer Eigenschaften trennen.

Während die nicht wiederverwendbare Fraktion 461 der Entsorgung zugeführt wird, wird die wiederverwendbare Fraktion 462 in der Konditioniervorrichtung 470 aufgereinigt und in eine für die erneute Verarbeitung günstige Form gebracht. Hierbei kann es sich beispielsweise um ein aufgespultes Filament 471 oder ein gleichmäßiges Granulat handeln.

Das entsprechend Konditionierte Material wird dann dem 3D-Drucksystem zugeführt, um bei der Herstellung neuer Adapter verwendet zu werden.

## Patentansprüche

1. Adapter zum Anschluss eines Endoskops (1,201,301,401) mit wenigstens einem Endoskopkanal (7,8) an ein Aufbereitungsgerät (210,310a,310b,310c,410) mit wenigstens einem Aufbereitungskanal (21,22), wobei der Adapter (29,129,250,350,450) ein Verbindungskanalsystem (45,145) aufweist, welches zur Verbindung des wenigstens einen Endoskopkanals (7,8) mit dem wenigstens einen Aufbereitungskanal (21,22) vorgesehen ist, um eine zuverlässige Aufbereitung des wenigstens einen Endoskopkanals (7,8) durch das Aufbereitungsgerät (210,310a,310b,310c,410) zu ermöglichen, wobei der Adapter (29,129,250,350,450) zumindest teilweise in einem 3D-Druckverfahren hergestellt ist, **dadurch gekennzeichnet, dass**
- der Adapter (29,129,250,350,450) zur Einmalverwendung vorgesehen ist, und
- dass der Adapter (29,129,250,350,450) ein Kupplungselement (131,132) zur Verbindung mit dem Aufbereitungsgerät (210,310a,310b,310c,410) aufweist, welches zum Lösen des Adapters (29,129,250,350,450) von dem Aufbereitungsgerät (210,310a,310b,310c,410) zerstört werden muss.

2. Adapter nach Anspruch 1, **dadurch gekennzeichnet, dass** der Adapter (29,129,250,350,450) einen das Verbindungskanalsystem (45,145) umfassenden Hauptkörper (30,130,251) aufweist, und dass der Hauptkörper (30,130,251) in einem 3D- Druckverfahren hergestellt ist.

3. Adapter nach Anspruch 1 oder 2, **dadurch gekennzeichnet, dass** der Adapter (29,129,250,350,450) Schlauchkupplungen (33,34,35,39,40,133,134,135,253) aufweist, und dass die Schlauchkupplungen (33,34,35,39,40,133,134,135,253) nicht in einem 3D-Druckverfahren hergestellt sind.

4. Adapter nach einem der Ansprüche 1 bis 3, **dadurch gekennzeichnet, dass** der Adapter Schläuche (36,37,38,136,137,138) umfasst, und dass die Schläuche (36,37,38,136,137,138) nicht in einem 3D-Druckverfahren hergestellt sind.

5. Adapter nach einem der Ansprüche 1 bis 4, **dadurch gekennzeichnet, dass** der Adapter (29,129,250,350,450) zumindest teilweise aus einem wiederverwendbaren Druckmaterial hergestellt ist.

6. System zur Aufbereitung von Endoskopen (1,201,301,401), umfassend:
- wenigstens ein Aufbereitungsgerät (210,310a,310b,310c,410) mit wenigstens einem Aufbereitungskanal (21,22),
- ein 3D-Drucksystem (240,340,440) zur Herstellung von Adaptern,
- eine Datenbank (230,330,430) zur Speicherung der Druckdaten für Adapter,
- ein Detektor (212,370,390) zur Identifikation eines aufzubereitenden Endoskops (1,201,301,401), sowie
- eine Steuerung (220,320,420),
wobei die Steuerung (220,320,420) dazu eingerichtet ist,
- mittels des Detektors (212,370,390) ein aufzubereitendes Endoskop (1,201,301,401) zu identifizieren,
- ein zur Aufbereitung des Endoskops (1,201,301,401) ausgewähltes Aufbereitungsgerät (210,310a,310b,310c,410) zu ermitteln,
- aus der Datenbank die 3D-Druckdaten eines Adapters (29,129,250,350,450) zu ermitteln, wobei der Adapter (29, 129, 250, 350, 450) zur Verbindung des aufzubereitenden Endoskops (1,201,301,401) in dem ausgewählten Aufbereitungsgerät (210,310a,310b,310c,410) geeignet ist und ein Verbindungskanalsystem (45, 145) aufweist, welches zur Verbindung des wenigstens einen Endoskopkanals (7, 8) mit dem wenigstens einen Aufbereitungskanal (21, 22) vorgesehen ist, um eine zuverlässige Aufbereitung des wenigstens einen Endoskopkanals (7, 8) durch das Aufbereitungsgerät (210, 310a, 310c, 410) zu ermöglichen, wobei der Adapter (29, 129, 250, 350, 450) zur Einmalverwendung vorgesehen ist,
- die ermittelten 3D-Druckdaten an das 3D-Drucksystem (240,340,440) zu übermitteln, und
- das 3D-Drucksystem (240,340,440) zur Herstellung des Adapters (29,129,250,350,450) zu veranlassen.

7. System nach Anspruch 6, **dadurch gekennzeichnet, dass** der Detektor (212) ausgeführt ist, ein aufzubereitendes Endoskop (201) zu identifizieren, wenn dieses in ein Aufbereitungsgerät (210) eingebracht wird.

8. System nach Anspruch 6, **dadurch gekennzeichnet, dass** der Detektor (370,390) ausgeführt ist, ein aufzubereitendes Endoskop (301) zu identifizieren, sobald dieses Endoskop (301) in einer Prozedur verwendet wird.

9. System nach einem der Ansprüche 6 bis 8, **dadurch gekennzeichnet, dass** das System weiterhin eine Einrichtung (460,470) zur Rückgewinnung von 3D-Druckmaterial aus gebrauchten Adaptern (450) umfasst.

10. Verfahren zum Herstellen eines Adapters gemäß eines der Ansprüche 1 bis 5 mit den Schritten:
- Bereitstellen eines Basismaterials zur Herstellung des Adapters (29,129,250,350,450), wobei das Basismaterial zur Durchführung eines 3D-Druckverfahrens geeignet ist,
- Bereitstellen von 3D-Druckdaten des Adapters (29,129,250,350,450),
- wenigstens teilweises Herstellen des Adapters (29,129,250,350,450) in einem 3D-Druckverfahren unter Verwendung des Basismaterials und der 3D-Druckdaten.

11. Verfahren nach Anspruch 10, **dadurch gekennzeichnet, dass** der Adapter (29,129,250,350,450) aus Kunststoff hergestellt wird.

12. Verfahren nach Anspruch 11, **dadurch gekennzeichnet, dass** der Kunststoff Polyactide (PLA) beinhaltet.

13. Verfahren nach Anspruch 11 oder 12, **dadurch gekennzeichnet, dass** der Kunststoff Acrylnitril-Butadien-Styrol (ABS) beinhaltet.

## Claims

1. Adapter for connecting an endoscope (1, 201, 301, 401) having at least one endoscope channel (7, 8) to a reprocessing device (210, 310a, 310b, 310c, 410) having at least one reprocessing channel (21, 22), wherein the adapter (29, 129, 250, 350, 450) has a connection channel system (45, 145), which is provided to connect the at least one endoscope channel (7, 8) to the at least one reprocessing channel (21, 22) to make reliable reprocessing of the at least one endoscope channel (7, 8) by way of the reprocessing device (210, 310a, 310b, 310c, 410) possible, wherein the adapter (29, 129, 250, 350, 450) is produced at least partially in a 3D printing method, **characterized in that**
- the adapter (29, 129, 250, 350, 450) is intended for single use, and
- **in that** the adapter (29, 129, 250, 350, 450) has a coupling element (131, 132) for connection to the reprocessing device (210, 310a, 310b, 310c, 410), which coupling element must be destroyed in order to remove the adapter (29, 129, 250, 350, 450) from the reprocessing device (210, 310a, 310b, 310c, 410).

2. Adapter according to Claim 1, **characterized in that** the adapter (29, 129, 250, 350, 450) has a main body (30, 130, 251), which comprises the connection channel system (45, 145), and **in that** the main body (30, 130, 251) is produced in a 3D printing method.

3. Adapter according to Claim 1 or 2, **characterized in that** the adapter (29, 129, 250, 350, 450) has hose couplings (33, 34, 35, 39, 40, 133, 134, 135, 253), and **in that** the hose couplings (33, 34, 35, 39, 40, 133, 134, 135, 253) are not produced in a 3D printing method.

4. Adapter according to one of Claims 1 to 3, **characterized in that** the adapter comprises hoses (36, 37, 38, 136, 137, 138), and **in that** the hoses (36, 37, 38, 136, 137, 138) are not produced in a 3D printing method.

5. Adapter according to one of Claims 1 to 4, **characterized in that** the adapter (29, 129, 250, 350, 450) is produced at least partially from a reusable printing material.

6. System for reprocessing endoscopes (1, 201, 301, 401), comprising:
- at least one reprocessing device (210, 310a, 310b, 310c, 410) having at least one reprocessing channel (21, 22),
- a 3D printing system (240, 340, 440) for producing adapters,
- a database (230, 330, 430) for storing the printing data for adapters,
- a detector (212, 370, 390) for identifying an endoscope (1, 201, 301, 401) to be reprocessed, and
- a controller (220, 320, 420),
wherein the controller (220, 320, 420) is configured for
- identifying an endoscope (1, 201, 301, 401) to be reprocessed using the detector (212, 370, 390),
- ascertaining a reprocessing device (210, 310a, 310b, 310c, 410) selected for reprocessing the endoscope (1, 201, 301, 401),
- ascertaining the 3D printing data of an adapter (29, 129, 250, 350, 450) from the database, wherein the adapter (29, 129, 250, 350, 450) is suitable for connecting the endoscope (1, 201, 301, 401) to be reprocessed in the selected reprocessing device (210, 310a, 310b, 310c, 410) and has a connection channel system (45, 145), which is provided for connecting the at least one endoscope channel (7, 8) to the at least one reprocessing channel (21, 22) to make reliable reprocessing of the at least one endoscope channel (7, 8) by way of the reprocessing device (210, 310a, 310c, 410) possible, wherein the adapter (29, 129, 250, 350, 450) is provided for single use,
- transmitting the ascertained 3D printing data to the 3D printing system (240, 340, 440), and
- causing the 3D printing system (240, 340, 440) to produce the adapter (29, 129, 250, 350, 450).

7. System according to Claim 6, **characterized in that** the detector (212) is embodied to identify an endoscope (201) to be reprocessed if said endoscope is introduced into a reprocessing device (210).

8. System according to Claim 6, **characterized in that** the detector (370, 390) is embodied to identify an endoscope (301) to be reprocessed as soon as said endoscope (301) is used in a procedure.

9. System according to one of Claims 6 to 8, **characterized in that** the system furthermore comprises an apparatus (460, 470) for recovering 3D printing material from used adapters (450).

10. Method for producing an adapter according to one of Claims 1 to 5, having the steps of:
- providing a base material for producing the adapter (29, 129, 250, 350, 450), wherein the base material is suitable for performing a 3D printing method,
- providing 3D printing data of the adapter (29, 129, 250, 350, 450),
- at least partially producing the adapter (29, 129, 250, 350, 450) in a 3D printing method using the base material and the 3D printing data.

11. Method according to Claim 10, **characterized in that** the adapter (29, 129, 250, 350, 450) is produced from plastic.

12. Method according to Claim 11, **characterized in that** the plastic contains polyactide (PLA).

13. Method according to Claim 11 or 12, **characterized in that** the plastic contains acrylonitrile butadiene styrene (ABS).

## Revendications

1. Adaptateur destiné au raccordement d'un endoscope (1, 201, 301, 401) comprenant au moins un canal d'endoscope (7, 8) à un appareil de retraitement (210, 310a, 310b, 310c, 410) comprenant au moins un canal de retraitement (21, 22), l'adaptateur (29, 129, 250, 350, 450) possédant un système de canal de liaison (45, 145) qui est conçu pour relier l'au moins un canal d'endoscope (7, 8) à l'au moins un canal de retraitement (21, 22) afin de rendre possible un retraitement fiable de l'au moins un canal d'endoscope (7, 8) par l'appareil de retraitement (210, 310a, 310b, 310c, 410), l'adaptateur (29, 129, 250, 350, 450) étant au moins partiellement fabriqué dans un procédé d'impression 3D, **caractérisé en ce que**
- l'adaptateur (29, 129, 250, 350, 450) est conçu pour un usage unique, et
- **en ce que** l'adaptateur (29, 129, 250, 350, 450) possède un élément de couplage (131, 132) destiné à la liaison avec l'appareil de retraitement (210, 310a, 310b, 310c, 410), lequel doit être détruit pour détacher l'adaptateur (29, 129, 250, 350, 450) de l'appareil de retraitement (210, 310a, 310b, 310c, 410).

2. Adaptateur selon la revendication 1, **caractérisé en ce que** l'adaptateur (29, 129, 250, 350, 450) possède un corps principal (30, 130, 251) qui comporte le système de canal de liaison (45, 145), et **en ce que** le corps principal (30, 130, 251) est fabriqué dans un procédé d'impression 3D.

3. Adaptateur selon la revendication 1 ou 2, **caractérisé en ce que** l'adaptateur (29, 129, 250, 350, 450) possède des coupleurs pour tuyau (33, 34, 35, 39, 40, 133, 134, 135, 253), et **en ce que** les coupleurs pour tuyau (33, 34, 35, 39, 40, 133, 134, 135, 253) ne sont pas fabriqués dans un procédé d'impression 3D.

4. Adaptateur selon l'une des revendications 1 à 3, **caractérisé en ce que** l'adaptateur comporte des tuyaux (36, 37, 38, 136, 137, 138), et **en ce que** les tuyaux (36, 37, 38, 136, 137, 138) ne sont pas fabriqués dans un procédé d'impression 3D.

5. Adaptateur selon l'une des revendications 1 à 4, **caractérisé en ce que** l'adaptateur (29, 129, 250, 350, 450) est au moins partiellement fabriqué à partir d'un matériau d'impression réutilisable.

6. Système de retraitement d'endoscopes (1, 201, 301, 401), comprenant :
- au moins un appareil de retraitement (210, 310a, 310b, 310c, 410) comprenant au moins un canal de retraitement (21, 22),
- un système d'impression 3D (240, 340, 440) destiné à fabriquer des adaptateurs,
- une base de données (230, 330, 430) destinée à mémoriser les données d'impression pour l'adaptateur,
- un détecteur (212, 370, 390) destiné à identifier un endoscope (1, 201, 301, 401) à retraiter et
- une commande (220, 320, 420),
la commande (220, 320, 420) étant conçue pour,
- au moyen du détecteur (212, 370, 390), identifier un endoscope (1, 201, 301, 401) à retraiter,
- déterminer un appareil de retraitement (210, 310a, 310b, 310c, 410) sélectionné pour le retraitement de l'endoscope (1, 201, 301, 401),
- déterminer les données d'impression 3D d'un adaptateur (29, 129, 250, 350, 450) à partir de la base de données, l'adaptateur (29, 129, 250, 350, 450) étant adapté pour relier l'endoscope (1, 201, 301, 401) à retraiter dans l'appareil de retraitement (210, 310a, 310b, 310c, 410) sélectionné et possédant un système de canal de liaison (45, 145) qui est conçu pour relier l'au moins un canal d'endoscope (7, 8) à l'au moins un canal de retraitement (21, 22) afin de rendre possible un retraitement fiable de l'au moins un canal d'endoscope (7, 8) par l'appareil de retraitement (210, 310a, 310c, 410), l'adaptateur (29, 129, 250, 350, 450) étant conçu pour un usage unique,
- communiquer les données d'impression 3D déterminées au système d'impression 3D (240, 340, 440) et
- amener le système d'impression 3D (240, 340, 440) à fabriquer l'adaptateur (29, 129, 250, 350, 450).

7. Système selon la revendication 6, **caractérisé en ce que** le détecteur (212) est exécuté pour identifier un endoscope (201) à retraiter lorsque celui-ci est introduit dans un appareil de retraitement (210).

8. Système selon la revendication 6, **caractérisé en ce que** le détecteur (370, 390) est exécuté pour identifier un endoscope (301) à retraiter dès que cet endoscope (301) est utilisé dans une procédure.

9. Système selon l'une des revendications 6 à 8, **caractérisé en ce que** le système comporte en outre un dispositif (460, 470) destiné à récupérer du matériau d'impression 3D à partir d'adaptateurs (450) usagés.

10. Procédé de fabrication d'un adaptateur selon l'une des revendications 1 à 5, comprenant les étapes suivantes :
- fourniture d'un matériau de base en vue de la fabrication de l'adaptateur (29, 129, 250, 350, 450), le matériau de base étant adapté à la mise en œuvre d'un procédé d'impression 3D,
- fourniture de données d'impression 3D de l'adaptateur (29, 129, 250, 350, 450),
- fabrication au moins partielle de l'adaptateur (29, 129, 250, 350, 450) dans un procédé d'impression 3D en utilisant le matériau de base et les données d'impression 3D.

11. Procédé selon la revendication 10, **caractérisé en ce que** l'adaptateur (29, 129, 250, 350, 450) est fabriqué à partir d'une matière plastique.

12. Procédé selon la revendication 11, **caractérisé en ce que** la matière plastique contient du polylactide (PLA).

13. Procédé selon la revendication 11 ou 12, **caractérisé en ce que** la matière plastique contient de l'acrylonitrile butadiène styrène (ABS).
